# EUROPEAN PATENT SPECIFICATION

(11) **EP 1 967 232 B1**
(45) Date of publication and mention of the grant of the patent: **28.12.2011**
(21) Application number: 06761453.7
(22) Date of filing: 17.07.2006
(51) Int. Cl.: A61N 7/00

(54) **SWINGING HIGH INTENSITY FOCUSED ULTRASOUND THERAPEUTIC APPARATUS AND MRI GUIDED HIGH INTENSITY FOCUSED ULTRASOUND THERAPEUTIC SYSTEM THEREOF**
SCHWINGENDES HOCHINTENSIVES FOKUSSIERTES THERAPEUTISCHES ULTRASCHALLGERÄT UND MRT-GEFÜHRTES HOCHINTENSIVES FOKUSSIERTES THERAPEUTISCHES ULTRASCHALLSYSTEM DAFÜR
APPAREIL THERAPEUTIQUE A ULTRASONS FOCALISE DE HAUTE INTENSITE D'OSCILLATION ET SYSTEME THERAPEUTIQUE A ULTRASONS FOCALISE DE HAUTE INTENSITE D'IMAGERIE GUIDEE PAR RESONANCE MAGNETIQUE ASSOCIE

(30) Priority: 27.12.2005 CN 200510132833
(43) Date of publication of application: 10.09.2008
(73) Proprietor: Chongqing Ronghai Medical Ultrasound Industry Ltd., Yubei District Chongqing 401121 (CN)
(72) Inventor: WANG, Long, Chongqing 401121 (CN); MU, Mu, Chongqing 401121 (CN); FU, Bing, Chongqing 401121 (CN); WANG, Hai, Chongqing 401121 (CN)
(74) Representative: von Kreisler Selting Werner
(86) International application number: PCT/CN2006/001713
(87) International publication number: WO 2007/073642

(56) References cited:
- EP-A- 0 373 967
- EP-A- 1 348 385
- WO-A-98/52465
- WO-A-2005/065409
- CN-A- 1 257 414
- US-A- 5 044 354
- US-A- 5 213 102
- US-A- 5 443 068
- US-A- 5 526 814
- US-A1- 2001 039 379
- US-A1- 2003 018 269
- US-A1- 2003 216 648
- US-A1- 2005 107 702
- US-A1- 2005 154 295

## Description

### TECHNICAL FIELD OF THE INVENTION

The present invention pertains to the technical field of ultrasound therapy, and relates to a swing type high-intensity focused ultrasound therapeutic apparatus and an MRI guided high-intensity focused ultrasound therapeutic system having such a swing type apparatus.

### BACKGROUND OF THE INVENTION

The focused ultrasound therapies in the prior art mainly use B-mode ultrasound apparatus to locate the region of the subject and to monitor the therapy. Such B-mode ultrasound apparatus has the following disadvantages on monitoring and treating: 1. B-mode ultrasound image is simply a flat image with a certain angle, so even though a 3-D ultrasound system is used, the visible area is still limited; 2. the ultrasound image is limited on the observation depth, the bone substances have a great effect on the image, so the tissue behind the bone can barely be displayed and there are artifacts. 3, Ultrasound images have poor capacity to identify the tissue boundary and particularly it is more difficult to identify small tumors and deep-bedded tumors.

Furthermore, MRI (Magnetic Resonance Imaging) apparatus is an important apparatus in biological and medical fields. It may apply appropriate gradients to the magnetic field so that the magnetic resonance signals can be acquired selectively, and the information is processed to obtain the tissue characteristics of each point and thereby the tissue can be imaged. The acquired Magnetic Resonance Image has a great ability to identify different tissues and can easily distinguish the normal tissue from the tumor tissue. It provides the tridimensional data of a certain volume so that a part of human body or even a full body can be imaged, therefore, MRI is very suitable to monitor in the high-intensity focused ultrasound therapies.

Japanese Patent No. 3322649 discloses a therapy system combining an MRI apparatus with a piece of ultrasound therapy equipment. Normally, the vertical moving distance under the treatment bed in the bore of MRI apparatus is about 140mm, thus, the ultrasound therapy equipment moves vertically therein for a treatment in quite a limited space Therefore, this therapy system employs MRI apparatus to determine the location of a tumor beforehand, and then the patient is moved out from the magnetic field of MRI and then is treated with ultrasound therapy. This therapy system needs repeated moves of patient and multiple locations. The locating system is complex and the location costs a long time.

US Patents No. 5275165 and No. 5443068 and No. 5526814 disclose a non-magnetic positioning apparatus of an ultrasound energy applicator operated in the bore of a magnetic field of MRI system with a purpose of avoiding any interference from the positioning apparatus to the magnetic field of MRI system. But the movement modes of the ultrasound transducer applicators in these positioning apparatus are quite limited and the requirements on treating tumors in multiple dimensions cannot be met.

In addition, US Patent No. 6148225 with assignee of Siemens Aktiengesellschaft discloses an ultrasound therapy apparatus, which has a high-frequency generator to generate electrical signals with different discrete frequency values that lie within a frequency band in chronological sequence. Whole multiples of the discrete frequency values do not lie in a second frequency band that corresponds to the reception band of a simultaneously operated diagnostic MRI apparatus. In this way, the ultrasound therapy apparatus does not disturb the magnetic field of the magnetic resonance apparatus when the ultrasound therapies are carried out during MRI monitoring.

The therapy system described in each patent as mentioned above is an ultrasound therapy system combined with an MRI system. These therapy systems mainly have the following disadvantages: 1) the vertical moving distance under the treatment bed in the bore of MRI apparatus is quite limited, so it is very difficult to place an ultrasound transducer requiring a multiple-dimensional movement under the treatment bed and it is more difficult to place a complex moving-locating mechanism, and the moving-locating mechanism placed under the treatment bed in the bore of MRI apparatus occupies the limited space for moving an ultrasound transducer, owing to which the treatment procedures are affected; 2) the moving-locating mechanism arranged in the bore of MRI apparatus may bring interference to MRI apparatus and accordingly the images of tissue acquired may not be correct, therefore, highly required non-magnetic designs and treatment on the ultrasound transducer and its moving-locating mechanism are needed for these systems, accordingly, the technical complexity and the cost of manufacturing these systems are increased; 3) the ultrasound transducer driven by the moving-locating mechanism can only move within a limited space and can not meet the requirements of treating tumors in multiple dimensions.

### SUMMARY OF THE INVENTION

Based on the above-mentioned disadvantages of the prior art, the technical problem is solved in the present invention by providing a swing type high-intensity focused ultrasound therapeutic apparatus, which has an ultrasound transducer that moves freely, and an MRI guided high-intensity focused ultrasound therapeutic system, which brings less interference to MRI apparatus and can acquire ultrasound images of good quality.

The technical solution for solving the problems is as follows: An MRI guided high-intensity focused ultrasound therapeutic system comprises an MRI apparatus which includes a bore and a first treatment bed (14) placed in the bore thereof, and high-intensity focused ultrasound therapeutic apparatus which includes an ultrasound transducer (1) and a moving-locating mechanism thereof, said moving-locating mechanism is a swing mechanism connected to the ultrasound transducer (1) by a supporting rod (2), and the moving-locating mechanism is placed outside of the bore of the MRI apparatus and said supporting rod (2) connected to said moving-locating mechanism can extend into the bore of the MRI apparatus.

The invention is defined by claim 1.

Said swing mechanism may comprise a Y-axis swing mechanism having a Y-axis swing center, a Y-axis swing rod connected to the Y-axis swing center, a Y-axis driving device for driving the Y-axis swing center to rotate and a supporting rod fixed on the Y-axis swing rod.

Preferably, said swing mechanism further comprises a Z-axis swing mechanism having a Z-axis swing center, a Z-axis driving device for driving the Z-axis swing center to rotate and a supporting rod fixed on the Z-axis swing center. Wherein, the Y-axis driving device and Z-axis driving device are motors and the Y-axis swing center and Z-axis swing center are synchronization belt-wheels connected respectively to Y and Z-axis driving device by a synchronization belt.

More preferably, in order to move the ultrasound transducer to a better appropriate location for a treatment, said swing mechanism further comprises a driving device to drive the supporting rod to autorotate along X-axis. The supporting rod is connected to the output shaft of said driving device.

More preferably, there is an X-axis motion structure under said swing mechanism. The X-axis motion structure comprises a threaded rod arranged in X direction, a base plate fixed on the nut of the threaded rod and a driving device to drive the threaded rod. Said swing mechanism is mounted on the base plate.

In order to drive the ultrasound transducer to traverse freely by the moving-locating mechanism, a sliding device is arranged under said base plate. The sliding device is a sliding wheel, preferably a universal wheel. When force is applied to the moving-locating mechanism, the whole moving-locating mechanism can move under the drive of the sliding wheel and the ultrasound transducer can move under the drive of the supporting rod. This additional device enables an operator to manually operate the ultrasound transducer during treatment and is more helpful for the operator to have clinical observations during treatment.

In the swing mechanism of the present invention, the swing angle in Y-axis or Z-axis of the supporting rod ranges from -40° to + 40°.

Furthermore, said swing mechanism also may only include the Z-axis swing mechanism, wherein, the Z-axis swing mechanism comprises a Z-axis swing center, a Z-axis swing rod connected to the Z-axis swing center, a Z-axis driving device for driving the Z-axis swing center to rotate and a supporting rod fixed on the Z-axis swing rod; or, said swing mechanism may only include the Y-axis swing mechanism.

The ultrasound transducer of the present invention may be placed in a container full of fluid. Said container is connected to the supporting rod and the container is full of fluid and said fluid may be the degassed water

In the therapy apparatus of the present invention, the supporting rod can move in all directions under the drive of the moving-locating mechanism system and the ultrasound transducer is connected to the supporting rod and thereby the ultrasound transducer can be accurately moved and located.

In the therapy apparatus of the present invention, the moving-locating mechanism is a swing mechanism, and when the supporting rod swings in Z-axis or Y-axis, the moving range of the driving device (which refers to a motor) of the whole moving-locating mechanism is quite limited or even none. Therefore, when this swing type high-intensity focused ultrasound therapeutic apparatus is used in conjunction with an ultrasound imaging and diagnosis apparatus (for example, an MRI apparatus), the electromagnetism of the motor almost has no effects on the imaging quality and accordingly, the interference due to the magnetic field of a motor in the moving-locating mechanism to the magnetic field of the ultrasound imaging and diagnosis apparatus can be overcome well. The moving-locating mechanism provides various kinds of movement modes and enables the medical staff to treat a patient in various kinds of body positions.

An MRI guided high-intensity focused ultrasound therapeutic system comprises an MRI apparatus, which includes a first treatment bed placed in the bore, and the above-mentioned swing type high-intensity focused ultrasound therapeutic apparatus, wherein the moving-locating mechanism is placed outside of the bore of the MRI apparatus and the supporting rod connected to said moving-locating mechanism can extend into the bore of the MRI apparatus.

Preferably, for convenient operations, the moving-locating mechanism is placed under the first treatment bed. The MRI apparatus further comprises a second treatment bed, on which a support is placed, and the moving-locating mechanism is placed on said support.

More preferably, the supporting rod may be made of non-magnetic materials or non-metal materials, accordingly the interference from high-intensity focused ultrasound therapeutic apparatus to the magnetic field of the MRI apparatus is further decreased and the negative influence on imaging quality can be avoided.

When the therapy system of the present invention is used in a treatment, a patient is placed on the second treatment bed. Said moving-locating mechanism is placed outside of the bore of the MRI apparatus and one end of the supporting rod connected to the ultrasound transducer extends into the bore of the MRI apparatus. In this way, the present invention makes the area applied with energy by ultrasound transducer intercrosses with the imaging and monitoring area of the MRI apparatus at the target area to be treated. Meanwhile, the therapy system of the present invention better integrates the MRI apparatus with the ultrasound therapeutic apparatus and increases the utilization ratio of equipment.

Due to the limited space in the bore of MRI apparatus and according to the needs of the clinical plan, the swing angle in Y-axis or Z-axis of the supporting rod ranges from -40° to + 40°. Under the circumstaces the above-mentioned swing angle range is ensured, the sizes of the supporting rod and the whole moving-locating mechanism are designed so as to guarantee that the supporting rod will not hit the Y-axis swing mechanism underneath or the treatment bed of the MRI apparatus.

The therapy system of the present invention comprises a swing type moving-locating mechanism. Comparing to a linear moving-locating mechanism, the swing type one has a smaller size and a relative larger swing angle. Therefore, for a swing type moving-locating mechanism and a linear moving mechanism with a same volume, the movement range of the swing type moving mechanism is larger. Meanwhile, because the spatial moving mechanism generally consists of multiple moving mechanisms in a single direction, such as a Y-axis, a Z-axis and etc., when a linear moving mechanism moves in a certain direction (for example, a Y-direction), the driving motors of other one-way moving mechanisms (for example, a Z-direction moving mechanism) always move in the same direction. Because the motor has electromagnetism, when the motor moves closer to the MRI apparatus, the electromagnetism of the motor will influence the imaging effects of MRI apparatus. The present invention has a swing mechanism. When the ultrasound transducer swings in Y-axis or Z-axis, each driving motor in other directions does not move. Even though some driving motors move with it, the move range of the motor is quite limited and accordingly the negative effect on the imaging effects of the MRI apparatus due to the electromagnetism of the motor is avoided maximally.

### BRIEF DESCRIPTION OF THE DRAWINGS

Fig.1 is a structural schematic diagram of a swing type high-intensity focused ultrasound therapeutic apparatus according to the present invention.
Fig.2 is a structural schematic diagram of an MRI guided high-intensity focused ultrasound therapeutic system according to the present invention.

Wherein: 1-Ultrasound transducer 2-Supporting rod 3, 4, 6, motor 5, 7-synchronization belt-wheel 9-Threaded rod 10-Linear guide set 11 - Sliding wheel 12 - Water bag 13 - Seal 14 - First treatment bed 15-Hole 16 - MRI apparatus 17 - Second treatment bed 18 - Support 19-Y-axis swing rod 20-Base plate 21 - ( MRI ) bore 22 - Foundation 23 - Support bracket

### DESCRIPTION OF THE PREFERRED EMBODIMENTS

The present invention is further described in detail in conjunction with the following embodiment and the accompanying drawings.

The present embodiment is an unrestrictive embodiment of the present invention.

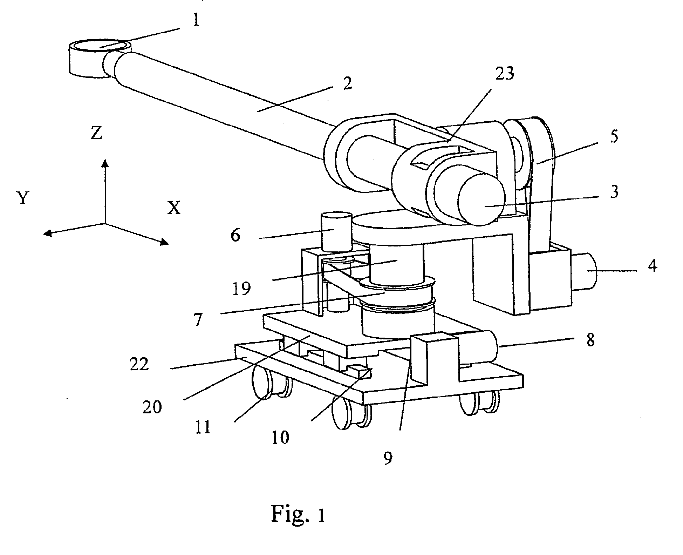

As shown in Fig. 1, the swing type high-intensity focused ultrasound therapeutic apparatus of the present invention comprises an ultrasound transducer 1 and a moving-locating mechanism for driving the ultrasound transducer 1 to move in multiple dimensions, wherein said moving-locating mechanism is a swing mechanism with a function of swinging and said swing mechanism is connected to a supporting rod 2, and the other end of the supporting rod 2 is connected to the ultrasound transducer 1.

In order to reduce the interference from the swing type high-intensity focused ultrasound therapeutic apparatus to the magnetic field of MRI apparatus and to avoid the negative effect on imaging quality, supporting rod 2 is made by non-magnetic materials or non-metal materials. In this embodiment, the material of the supporting rod is poly phenylene sulfide (PPS).

The ultrasound transducer 1 is placed in an open fluid container, i.e. a water bag 12, and the supporting rod 2 is connected to water bag 12 through a flexible seal 13, wherein water bag 12 is full of fluid and the fluid is degassed water in this embodiment.

There are two sets of swing mechanism in this embodiment, i.e. a Y-axis swing mechanism and a Z-axis swing mechanism, which drive the ultrasound transducer 1 to swing in Y-direction and Z-direction.

The Y-axis swing mechanism comprises a Y-axis swing center, a Y-axis swing rod 19 connected to the Y-axis swing center, a Y-axis driving device for driving the Y-axis swing center to rotate. In this embodiment, the Y-axis driving device is a motor, i.e., a motor 6, and the Y-axis swing center is a synchronization belt-wheel 7 connected to the motor 6 by a synchronization belt.

Z-axis swing mechanism comprises a Z-axis swing center, a Z-axis driving device for driving the Z-axis swing center to rotate. In this embodiment, the Z-axis driving device is a motor, i.e., a motor 4, and the Z-axis swing center is a synchronization belt-wheel 5 connected to the motor 4 by a synchronization belt.

The shaft of synchronization belt-wheel 5 is rigidly connected to support bracket 23. The supporting rod 2 is rotatably connected to support bracket 23. In this embodiment, the driving device for driving the supporting rod 2 to autorotate along X-axis is a motor, i.e. a motor 3, which is rigidly connected to support bracket 23. The output shaft of that motor is horizontally and rigidly connected to the supporting rod 2. The rotation of the axis of the motor 3 drives supporting rod 2 to rotate.

There is an X-axis moving mechanism under said Y-axis swing mechanism. The X-axis moving mechanism comprises a threaded rod 9 arranged in X direction, a base plate 20 fixed on the nut of the threaded rod and a driving device, i.e. a motor 8 for driving the threaded rod 9. Said Y-axis swing mechanism is mounted on the base plate 20. Because there is a guide slot under the base plate 20 and the linear guide set 10 on the foundation 22 is placed in that slot, the swing mechanism can move to and fro linearly in X direction.

There is a sliding device under the foundation 22, i.e. the sliding wheel 11 on the foundation 22. In this embodiment, the sliding wheel 11 is a universal wheel.

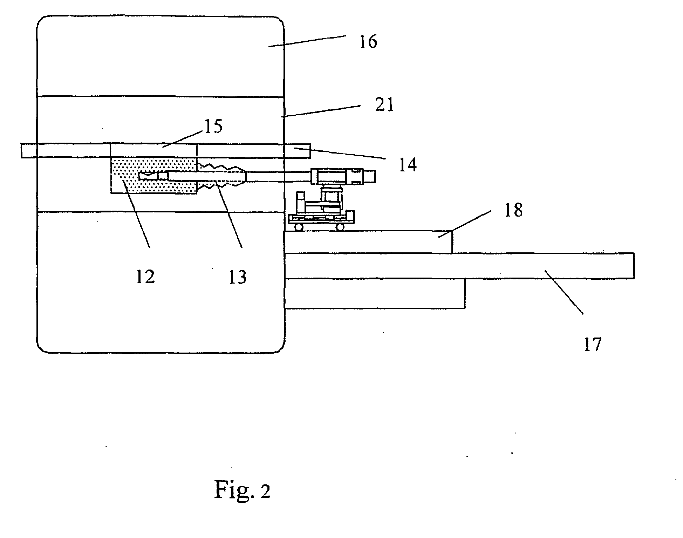

As shown in Fig. 2, there is a first treatment bed 14 and a second treatment bed 17 in the bore 21 of the MRI apparatus 16, wherein the first treatment bed 14 is above the second treatment bed 17, and a support 18 fixed on the second treatment bed 17 is used to support the swing type high-intensity focused ultrasound therapeutic apparatus of the present invention and said therapeutic apparatus is fixed on the support 18. In this way, an MRI guided high-intensity focused ultrasound therapeutic system is formed. In the treatment, the supporting rod 2 can extend into the bore 21 of the MRI apparatus and is located under the hole 15 of the first treatment bed 14. When a treatment is not needed, the supporting rod 2 drives the ultrasound transducer to withdraw from the bore 21 of the MRI apparatus 16 and the whole swing type high-intensity focused ultrasound therapeutic apparatus can be placed in the bore 21 of the MRI apparatus 16 for saving the space.

During the treatment, the patient is placed on the first treatment bed 14 at first and the diseased part to be exposed by ultrasound is aimed at the hole 15, then the end of supporting rod 2 connected to ultrasound transducer 1 extends into the bore 21 of the MRI apparatus 16 and the ultrasound transducer 1 is positioned under the first treatment bed 14 by the moving-locating mechanism- The whole moving-locating mechanism is fixed on the support 18 which is outside of the bore of the MRI apparatus, and through movements in multiple dimensions, the area applied with energy by ultrasound transducer 1 intercrosses with the imaging and monitoring area of MRI apparatus 16 at the target area to be treated.

When the ultrasound transducer I needs to move in Y-axis, in Y-axis direction, the output shaft of the motor 6 drives the synchronization belt-wheel 7 to rotate along Z-axis and the synchronization belt-wheel 7 drives the whole mechanism fixed on it to swing in Y direction. The motor 3 and the motor 4 also swing in a limited move range along with the mechanism fixed on the synchronization belt-wheel 7, but the motor 8 and the motor 6 do not move.

When the ultrasound transducer I needs to move in Z-axis, the output shaft of the motor 4 drives the synchronization belt-wheel 5 to rotate along Y-axis and the synchronization belt-wheel 5 drives the supporting rod 2 to swing in Z direction. The motor 3 swings in a limited move range along with the supporting rod 2, but the motor 6, the motor 8 and the motor 4 do not move.

Because the motor moves in quite a limited move range, when the therapeutic apparatus of the present invention is used in conjunction with the MRI apparatus 16, the magnetic field of the motor has few effects on the magnetic field of the MRI apparatus.

When ultrasound transducer 1 needs to move in X-axis, driven by the motor 8, the nut of the threaded rod drives the base plate 20 and the swing mechanism thereon to move linearly in X-axis along the linear guide set 10.

When the angle of the ultrasound transducer I itself needs to be changed, driven by the motor 3, the supporting rod 2 drives the ultrasound transducer 1 to autorotate along X-axis.

When the ultrasound transducer 1 needs to be operated manually, force can be applied to the foundation 22 by hand and the universal wheel under the foundation 22 can slide freely in all directions.

## Claims

1. An MRI guided high-intensity focused ultrasound therapeutic system comprising an MRI apparatus including a bore and a first treatment bed (14) placed in the bore thereof, and a high-intensity focused ultrasound therapeutic apparatus including an ultrasound transducer (1) and a moving-locating mechanism thereof the moving-locating mechanism being a swing mechanism **characterized by** said swing mechanism being placed outside of the bore of the MRI apparatus and connected to the ultrasound transducer (1) by a supporting rod (2), one end of said supporting rod (2) being connected to said swing mechanism, the other end of said supporting rod (2) being connected to the ultrasound transducer (1), and the end of said supporting rod (2) connected to the ultrasound transducer (1) extending into the bore of the MRI apparatus.

2. The MRI guided high-intensity focused ultrasound therapeutic system as claimed in claim 1, **characterized by** said swing mechanism comprising a Y-axis swing mechanism having a Y-axis swing center, a Y-axis swing rod (19) connected to the Y-axis swing center, a Y-axis driving device for driving the Y-axis swing center to rotate and the supporting rod (2) fixed on the Y-axis swing rod.

3. The MRI guided high-intensity focused ultrasound therapeutic system as claimed in claim 2, **characterized by** said swing mechanism further comprising a Z-axis swing mechanism having a Z-axis swing center, a Z-axis driving device for driving the Z-axis swing center to rotate and the supporting rod (2) fixed on the Z-axis swing center.

4. The MRI guided high-intensity focused ultrasound therapeutic system as claimed in claim 3, **characterized by** the Y-axis and Z-axis driving devices being motors and the Y-axis and Z-axis swing centers being synchronization belt-wheels respectively connected to Y and Z-axis driving devices by a synchronization belt.

5. The MRI guided high-intensity focused ultrasound therapeutic system as claimed in claim 3, **characterized by** the swing angle in Y-axis or Z-axis of the supporting rod ( 2 ) ranging from -40° to + 40°.

6. The MRI guided high-intensity focused ultrasound therapeutic system as claimed in claim 3, **characterized by** said swing mechanism further comprising a driving device to drive the supporting rod to autorotate along X-axis, the supporting rod (2) being connected to the output shaft of said driving device.

7. The MRI guided high-intensity focused ultrasound therapeutic system as claimed in any one of claims 2 to 6, **characterized by** an X-axis moving mechanism being set under said swing mechanism; the X-axis moving mechanism comprising a threaded rod (9) arranged in X direction, a base plate (20) fixed on the nut of the threaded rod and a driving device for driving the threaded rod (9); and said swing mechanism being mounted on the base plate (20).

8. The MRI guided high-intensity focused ultrasound therapeutic system as claimed in claim 7, **characterized by** a sliding device being connected under said base plate.

9. An MRI guided high-intensity focused ultrasound therapeutic system as claimed in claim 1, **characterized by** the moving-locating mechanism being placed under the first treatment bed (14), the MRI apparatus (16) further comprising the second treatment bed (17), on which a support (18) is fixed, and the moving-locating mechanism being placed on said support (18).

## Patentansprüche

1. MRT-geführtes hochintensives fokussiertes therapeutisches Ultraschallsystem mit einem MRT-Gerät, welches eine Öffnung und ein in der Öffnung angeordnetes erstes Behandlungsbett (14) aufweist, und einem hochintensiven fokussierten therapeutischen Ultraschallgerät mit einem Ultraschallwandler (1) und einem Bewegungs-/Positionierungsmechanismus desselben, wobei es sich bei dem Bewegungs-/Positionierungsmechanismus um einen Schwingmechanismus handelt, **dadurch gekennzeichnet, dass** der Schwingmechanismus außerhalb der Öffnung des MRT-Geräts angeordnet ist und mit dem Ultraschallwandler (1) durch eine Stützstange (2) verbunden ist, wobei ein Ende der Stützstange (2) mit dem Schwingmechanismus verbunden ist, während das andere Ende der Stützstange (2) mit dem Ultraschallwandler (1) verbunden ist, und wobei das mit dem Ultraschallwandler (1) verbundene Ende der Stützstange (2) sich in die Öffnung des MRT-Geräts erstreckt.

2. MRT-geführtes hochintensives fokussiertes therapeutisches Ultraschallsystem nach Anspruch 1, **dadurch gekennzeichnet, dass** der Schwingmechanismus einen Y-Achsen-Schwingmechanismus mit einem Y-Achsen-Schwingmittelpunkt, einer mit dem Y-Achsen-Schwingmittelpunkt verbundenen Y-Achsen-Schwingstange (19), einer Y-Achsen-Antriebsvorrichtung zum drehenden Antreiben des Y-Achsen-Schwingmittelpunkts und der an der Y-Achsen-Schwingstange befestigten Stützstange (2).

3. MRT-geführtes hochintensives fokussiertes therapeutisches Ultraschallsystem nach Anspruch 2, **dadurch gekennzeichnet, dass** der Schwingmechanismus ferner einen Z-Achsen-Schwingmechanismus mit einem Z-Achsen-Schwingmittelpunkt, einer Z-Achsenantriebsvorrichtung zum drehenden Antreiben des Z-Achsen-Schwingmittelpunkts und der an dem Z-Achsen-Schwingmittelpunkt befestigten Stützstange (2).

4. MRT-geführtes hochintensives fokussiertes therapeutisches Ultraschallsystem nach Anspruch 3, **dadurch gekennzeichnet, dass** die Y-Achsen- und die Z-Achsen-Antriebsvorrichtungen Motoren sind und die X-Achsen- und Y-Achsen-Schwingmittelpunkte Synchronriemenscheiben sind, die jeweils über einen Synchronriemen mit den Y- und Z-Achsen-Antriebsvorrichtungen verbunden sind.

5. MRT-geführtes hochintensives fokussiertes therapeutisches Ultraschallsystem nach Anspruch 3, **dadurch gekennzeichnet, dass** der Schwingwinkel in der Y-Achse oder der Z-Achse der Stützstange (2) von -40° bis +40° reicht.

6. MRT-geführtes hochintensives fokussiertes therapeutisches Ultraschallsystem nach Anspruch 3, **dadurch gekennzeichnet, dass** der Schwingmechanismus ferner eine Antriebsvorrichtung aufweist, um die Stützstange zur Autorotation um die X-Achse anzutreiben, wobei die Stützstange (2) mit der Abtriebswelle der Antriebsvorrichtung verbunden ist.

7. MRT-geführtes hochintensives fokussiertes therapeutisches Ultraschallsystem nach einem der Ansprüche 2 bis 6, **gekennzeichnet durch** einen X-Achsen-Bewegungsmechanismus, der unter dem Schwingmechanismus angeordnet ist; wobei der X-Achsen-Bewegungsmechanismus eine in X-Richtung angeordnete Gewindestange (9), eine an der Mutter der Gewindestange befestigte Basisplatte (20) und eine Antriebsvorrichtung zum Antreiben der Gewindestange (9) aufweist; und wobei der Schwingmechanismus an der Basisplatte (20) angebracht ist.

8. MRT-geführtes hochintensives fokussiertes therapeutisches Ultraschallsystem nach Anspruch 7, **gekennzeichnet durch** eine unter der Basisplatte verbundene Gleitvorrichtung.

9. MRT-geführtes hochintensives fokussiertes therapeutisches Ultraschallsystem nach Anspruch 1, **dadurch gekennzeichnet, dass** der Bewegungs-/Positionierungsmechanismus unter dem ersten Behandlungsbett (14) angeordnet ist, wobei das MRT-Gerät (16) ferner das zweite Behandlungsbett (17) aufweist, an welchem eine Stütze (18) befestigt ist, und wobei der Bewegungs-/Positionierungsmechanismus auf der Stütze (18) platziert ist.

## Revendications

1. Système thérapeutique à ultrasons focalisés de haute intensité d'imagerie guidée par résonance magnétique comprenant un appareil IRM avec une ouverture et un premier lit de traitement (14) placé dans ladite ouverture, et un appareil thérapeutique à ultrasons focalisés de haute intensité comportant un transducteur à ultrasons (1) et un mécanisme de déplacement/positionnement associé, ledit mécanisme de déplacement/positionnement étant un mécanisme d'oscillation, **caractérisé en ce que** ledit mécanisme d'oscillation est placé hors de ladite ouverture de l'appareil IRM et est connecté audit transducteur à ultrasons (1) par une tige de support (2), l'une des extrémités de ladite tige de support (2) étant connectée audit mécanisme d'oscillation, l'autre extrémité de ladite tige de support (2) étant connectée audit transducteur à ultrasons (1), et ladite extrémité de ladite tige de support (2) connectée audit transducteur à ultrasons (1) s'étendant dans ladite ouveture dudit appareil IRM.

2. Système thérapeutique à ultrasons focalisés de haute intensité d'imagerie guidée par résonance magnétique selon la revendication 1, **caractérisé en ce que** ledit mécanisme d'oscillation comprend un mécanisme d'oscillation selon l'axe Y avec un centre d'oscillation selon l'axe Y, une tige d'oscillation selon l'axe Y (19) connectée au centre d'oscillation selon l'axe Y, un dispositif d'entrainement pour entrainer en rotation ledit centre d'oscillation selon l'axe Y et ladite tige de support (2) fixée à ladite tige d'oscillation selon l'axe Y.

3. Système thérapeutique à ultrasons focalisés de haute intensité d'imagerie guidée par résonance magnétique selon la revendication 2, **caractérisé en ce que** ledit mécanisme d'oscillation comprend en outre un mécanisme d'oscillation selon l'axe Z avec un centre d'oscillation selon l'axe Z, un dispositif d'entrainement selon l'axe Z destiné à entrainer en r rotation ledit centre d'oscillation selon l'axe Z et ladite tige de support (2) fixée au centre d'oscillation selon l'axe Z.

4. Système thérapeutique à ultrasons focalisés de haute intensité d'imagerie guidée par résonance magnétique selon la revendication 3, **caractérisé en ce que** lesdits dispositifs d'entrainement selon l'axe Y et l'axe Z sont des moteurs et lesdits centres d'oscillation selon l'axe Y et l'axe Z sont des roues de courroie de synchronisation respectives, connectées à des dispositifs d'entrainement selon l'axe Y et l'axe Z par une courroie de synchronisation.

5. Système thérapeutique à ultrasons focalisés de haute intensité d'imagerie guidée par résonance magnétique selon la revendication 3, **caractérisé en ce que** l'angle d'oscillation selon l'axe Y ou l'axe Z de ladite tige de support (2) va de -40° à +40°.

6. Système thérapeutique à ultrasons focalisés de haute intensité d'imagerie guidée par résonance magnétique selon la revendication 3, **caractérisé en ce que** ledit mécanisme d'oscillation comprend en outre un dispositif d'entrainement pour entrainer ladite tige de support en autorotation selon l'axe X, ladite tige de support (2) étant connectée à l'arbre de sortie dudit dispositif d'entraînement.

7. Système thérapeutique à ultrasons focalisés de haute intensité d'imagerie guidée par résonance magnétique selon l'une des revendications 2 à 6, **caractérisé par** un mécanisme de déplacement selon l'axe X placé dessous ledit mécanisme d'oscillation; ledit mécanisme de déplacement selon l'axe X comprenant une tige à filetage (9) disposée selon la direction X, une plaque de base (20) fixée à l'écrou de ladite tige à filetage et un dispositif d'entrainement pour entrainer ladite tige à filetage (9); et ledit mécanisme d'oscillation étant fixé sur ladite plaque de base (20).

8. Système thérapeutique à ultrasons focalisés de haute intensité d'imagerie guidée par résonance magnétique selon la revendication 7, **caractérisé en ce qu'**un dispositif de glissement est connecté dessous ladite plaque de base.

9. Système thérapeutique à ultrasons focalisés de haute intensité d'imagerie guidée par résonance magnétique selon la revendication 1, **caractérisé en ce que** ledit mécanisme de déplacement/positionnement est disposé dessous ledit premier lit de traitement (14), ledit appareil IRM (16) comprenant en outre un deuxième lit de traitement (17) auquel est fixé un support (18), et que ledit mécanisme de déplacement/positionnement est disposé sur ledit support (18).
